**Europäisches Patentamt**

**European Patent Office**

**Office européen des brevets**

(11) Veröffentlichungsnummer: **0 100 924**
**B1**

# EUROPÄISCHE PATENTSCHRIFT

(45) Veröffentlichungstag der Patentschrift:
**22.06.88**

(51) Int. Cl.⁴: **A 61 F 5/47**

(21) Anmeldenummer: **83107054.5**

(22) Anmeldetag: **19.07.83**

(54) **Intrauterinpessar.**

(30) Priorität: **31.07.82 DE 3228704**

(43) Veröffentlichungstag der Anmeldung:
**22.02.84 Patentblatt 84/8**

(45) Bekanntmachung des Hinweises auf die Patenterteilung:
**22.06.88 Patentblatt 88/25**

(84) Benannte Vertragsstaaten:
**AT BE CH DE FR GB IT LI LU NL SE**

(56) Entgegenhaltungen:
**EP - A - 0 044 268**
**CH - A - 549 998**
**DE - A - 2 755 179**
**DE - A - 2 913 036**
**DE - A - 3 047 208**
**GB - A - 1 288 822**
**US - A - 3 598 115**
**US - A - 3 820 535**
**US - A - 3 954 103**

(73) Patentinhaber: **Fundatech S.A., 1,rue Robert-de-Traz Case postale 29 Champel, CH-1211 Genève - 12 (CH)**

(72) Erfinder: **Bauer, Hans Alfred, Dr. med, Werkstrasse 32, D-8523 Baiersdorf (DE)**

(74) Vertreter: **Böhme, Volker, Dr. et al, Patentanwälte Dr. Böhme, Kessel, Böhme Karolinenstrasse 27, D-8500 Nürnberg (DE)**

**Beschreibung**

Die Erfindung betrifft ein Intrauterinpessar mit einem im Cavum Ionen abgebenden länglichen Teil und einem an diesem vorgesehenem Befestigungsmittel zur Halterung im Cavum.

Bei einem bekannten (US-A-3 820 535) Intrauterinpessar dieser Art ist das Befestigungsmittel ein Kreuz, das zwei Arme mit Fäden aufweist. Die Festlegung dieses Intrauterinpessars im Cavum macht sich zunutze, dass sich das Cavum von der Vagina in Richtung zum Fundus im Querschnitt erweitert und in die beiden Eileiter übergeht. Die Arme ragen seitlich in Erweiterungen des Cavums. Diese Art der Festlegung ist bei der Einbringung wegen der vier Arme umständlich und nicht in allen Fällen ausreichend sicher, weil z.B. das Pessar in seiner Grösse nicht genau genug an die Abmessungen des Cavums angepasst ist oder die Motorik des Gebärmuttermuskels allzu stark ist.

Es ist auch bereits ein Intrauterinpessar bekannt (US-A-3 598 115), bei dem das Befestigungsmittel ein mit Spitze versehener Haken zur Verankerung im Fundus des Gebärmuttermuskels ist. Der Haken trägt einen Schaft mit seitlichen Flügeln, die in die seitlichen Erweiterungen des Cavums ragen. Die Motorik des Gebärmuttermuskels kann sehr gut auf die Flügel und den Schaft übertragen werden und damit an dem Haken angreifen, der relativ klein ausgebildet ist und deshalb den Muskel stark belastet.

Es ist schliesslich die Verankerung von Herzschrittmacherelektroden mittels einer mit Spitze versehenen Wendel bekannt (EP-A-0 044 268).

Aufgabe der Erfindung ist es daher, ein Intrauterinpessar der eingangs genannten Art zu schaffen, bei dem durch geeignete Gestaltung des Befestigungsmittels eine das längliche Teil berücksichtigende verbesserte Festlegung des Pessars im Cavum bzw. eine vereinfachte Einbringung sowie eine Minimierung der Expulsionsrate durch einen stets sicheren Sitz ermöglicht ist. Das erfindungsgemässe Pessar ist, diese Aufgabe lösend, dadurch gekennzeichnet, dass das Befestigungsmittel eine mit Spitze versehene Wendel zur Verankerung im Fundus des Gebärmuttermuskels ist.

Durch die Kombination von Wendel und länglichem Teil werden die auf den Gebärmuttermuskel ausübbaren Zugkräfte minimiert und die Haltekräfte maximiert, weil die Motorik des Gebärmuttermuskels nur wenig oder gar nicht an dem länglichen Teil angreifen kann und die Wendel mit einer sehr grossen Fläche im Fundus des Gebärmuttermuskels abgestützt ist. Nachdem das Problem der sicheren Lagerung von Intrauterinpessaren zu einer grossen Vielzahl von Befestigungsteilen geführt hat, die alle auf einer Verbreiterung im Cavum beruhen, löst die Erfindung dieses Problem auf überraschend einfache Weise. Da der erfindungsgemässe Pessar stets sicher sitzt, ist ein Kontrollfaden in der Vagina überflüssig, weshalb durch den Faden bedingte Infektionen entfallen.

Wenn der Intrauterinpessar rein metallisch ist, ist jeglicher Kunststoff im Cavum vermieden. Es tritt keine Pathogenisierung apathogener Keime und keine Resistenzbildung durch porösen Kunststoff auf.

Die Wendel lässt sich besonders einfach in den Muskel einbringen, da z.B. keine Hohlnadel als Hilfsmittel nötig ist. Die Lage der Wendelspitze lässt sich durch Gegendrehen sofort korrigieren, wenn die Wendelspitze z.B. durch den Muskel hindurchgetreten ist.

Im übrigen hat der erfindungsgemässe Pessar folgende Vorteile: Es gibt nach exakter Einlage keine spontane Expulsionsrate. Das relative Missverhältnis zwischen Cavum uteri und Intrauterinpessar ist vermieden. Blutungen durch mechanische Schleimhautverletzungen sind vermieden. Bei voller Harnblase sind Muskelstärke des Fundus und Lagekontrolle des Pessars mittels Ultraschall überwachbar. Der erfindungsgemässe Pessar ist im Röntgenbild vollständig sichtbar. Die Komplikationsrate ist unabhängig von den Gebärmuttermuskel- und den Cavum-Verhältnissen gleich niedrig. Der Pessar reagiert bei einem motorisch aktiven uterus rein passiv.

Besonders zweckmässig und vorteilhaft ist es, wenn mit Abstand von der Spitze der Verankerung ein Querstück vorgesehen ist. Das Querstück verhindert ein allzu tiefes Eindringen der Verankerung in den Muskel und stellt einen Anschlag dar.

Besonders zweckmässig und vorteilhaft ist es auch, wenn die Wendel Vertiefungen oder Erhebungen aufweist. Dies verbessert den Sitz der Wendel im Muskel.

Die Erfindung befasst sich auch mit einem Einsetz-Werkzeug für das Intrauterinpessar, das als längliches Röhrchen ausgebildet ist, in das das Intrauterinpessar einsetzbar ist und das mit Mitteln zum Austreiben des Pessars versehen ist. Dieses Einsetz-Werkzeug ist dadurch gekennzeichnet, dass das Intrauterinpessar samt Wendel in dem Röhrchen untergebracht ist und ein Querstück aufweist, das in Schlitze des Röhrchens eingreift. Beim Einschrauben wird die Wendel von alleine aus dem Röhrchen heraus bewegt.

Das an der Wendel vorgesehene Ionen abgebende längliche Teil ist z.B. eine Drahtwendel, deren Windungen dicht aneinanderliegen. Ein längliches Kupferteil, das koaxial mit der Verankerung verläuft, lässt sich besonders einfach durch den Gebärmutterhalskanal hindurchbringen. Auch lässt sich das längliche Kupferteil in ein Werkzeug einlegen, das verwendet wird, um die Wendel im Muskel anzubringen.

Das an der verankernden Wendel vorgesehene Ionen abgebende Teil ist z.B. als Kette ausgebildet. Da die Kette bei einem Lagewechsel der Schwerkraft folgt, erfolgt eine ständige Selbstreinigung der Metalloberfläche durch Reiben und Gleiten über Schleimhäute. Wegen dieser Selbstreinigung sind eine verlängerte Liegezeit des Pessars und eine ständig grosse Ionenabgabe ermöglicht.

In der Zeichnung ist eine bevorzugte Ausführungsform der Erfindung dargestellt und zeigt

Fig. 1 eine Seitenansicht eines Intrauterinpessars in einem im Schnitt dargestellten Werkzeug zum Einsetzen des Pessars,

Fig. 2 den Intrauterinpessar gemäss Fig. 1 in einer gegenüber Fig. 1 um 90° gedrehten Stellung und

Fig. 3 einen Schnitt gemäss Linie III–III in Fig. 1.

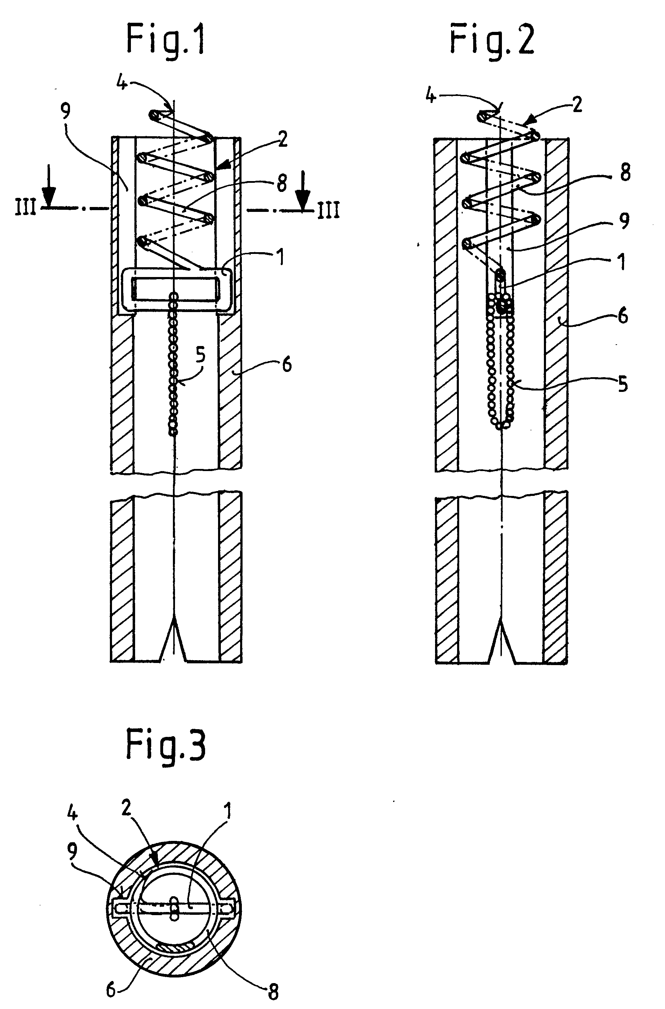

Der Pessar gemäss Zeichnung besitzt ein balkenartiges Querstück 1, das die Basis einer Verankerung 2 ist, die von einer schraubenartigen Wendel 8 gebildet ist, deren Windungen Abstand voneinander besitzen. Die Wendel 8 besteht aus Metall und besitzt am freien Ende eine Spitze 4. Ein an der hinteren Seite des Querstückes 1 angebrachtes längliches Kupferteil 5 ist von zwei Strängen einer Kette gebildet, die von ineinandergreifenden Ringgliedern gebildet ist. Die Ketten sind in der Zeichnung jeweils kürzer dargestellt als sie in Wirklichkeit in Relation zur Verankerung sind. Zum Beispiel ist die Kette gemäss Fig. 2 in Relation zur Länge der Verankerung etwa zehnmal so lang wie dargestellt.

Der Pessar ist in einem Röhrchen 6 untergebracht, das am vorderen Ende mit zwei Schlitzen 9 versehen ist, in die die beiden Enden des balkenartigen Querstückes 1 eingreifen. Das Röhrchen 6 dient nicht nur dazu, den Pessar durch die Vagina an den Gebärmuttermuskel heranzuführen, sondern auch dazu, die Wendel in den Muskel einzuschrauben. Beim Einschrauben wird die Wendel von alleine aus dem Röhrchen herausbewegt.

Die Verankerung 2 und/oder das Ionen abgebende Teil 5 bestehen z.B. aus Stahl, Kupfer, Silber, Gold und/oder Platin. Das Werkzeug 7 ist, wie gezeigt, in der Regel ein längliches, schlankes Röhrchen, das den Pessar aufnimmt, z.B. aus Kunststoff besteht, und Mittel aufweist, die an dem Querstück 1 angreifen, um die Verankerung aus dem Röhrchen in den Muskel zu treiben. Die Verankerung, die ein drahtähnliches längliches hintergreifendes Stück ist, weist verteilt Vertiefungen oder Erhebungen in der Oberfläche auf, um ein unerwünschtes Lösen aus dem Muskel, z.B. ein Zurückdrehen der Wendel zu verhindern.

## Patentansprüche

1. Intrauterinpessar mit einem im Cavum Ionen abgebenden, länglichen Teil und einem an diesem vorgesehenen Befestigungsmittel zur Halterung im Cavum, dadurch gekennzeichnet, dass das Befestigungsmittel eine mit Spitze (4) versehene Wendel (8) zur Verankerung im Fundus des Gebärmuttermuskels ist.

2. Intrauterinpessar nach Anspruch 1, dadurch gekennzeichnet, dass mit Abstand von der Spitze (4) ein Querstück (1) vorgesehen ist.

3. Intrauterinpessar nach Anspruch 1 oder 2, dadurch gekennzeichnet, dass die Wendel (8) Vertiefungen oder Erhebungen aufweist.

4. Einsetz-Werkzeug für das Intrauterinpessar nach Anspruch 1, 2 oder 3, das als längliches Röhrchen ausgebildet ist, in das das Intrauterinpessar einsetzbar ist und das mit Mitteln zum Austreiben des Pessars versehen ist, dadurch gekennzeichnet, dass das Intrauterinpessar samt Wendel (8) in dem Röhrchen (6) untergebracht ist und ein Querstück (1) aufweist, das in Schlitze (9) des Röhrchens eingreift.

## Claims

1. An intrauterine device with a longitudinal member which gives off ions in the cavum and a securing means to hold the said member in the cavum, characterised in that the securing means has a helix (8) provided with a point (4) for anchorage in the fundus of the uterine muscle.

2. An intrauterine device according to claim 1, characterised in that a transverse member (1) is provided at a distance from the point (4).

3. An intrauterine device according to claim 1 or 2, characterised in that the helix (8) has recesses and raised portions.

4. Device for inserting the intrauterine device according to claim 1, 2 or 3, which is formed as a longitudinal tube in which the intrauterine device is insertable and which is provided with means for removing the intrauterine device, characterised in that the intrauterine device is accommodated with the helix (8) in the tube (6) and has a transverse member which engages in slots (9) in the tube.

## Revendications

1. Stérilet comportant une partie de forme allongée, dégageant des ions dans la cavité utérine, et un moyen de fixation prévu sur cette partie et servant au maintien en place dans la cavité utérine, caractérisé en ce que le moyen de fixation est un élément hélicoïdal (8) avec une pointe (4) pour l'ancrage dans le fond du muscle utérin.

2. Stérilet selon la revendication 1, caractérisé en ce qu'il comporte un élément transversal (1) à distance de la pointe (4).

3. Stérilet selon la revendication 1 ou 2, caractérisé en ce que l'élément hélicoïdal (8) présente des creux ou des saillies.

4. Instrument d'insertion pour le stérilet selon la revendication 1, 2 ou 3, qui est réalisé comme un petit tube de forme allongée, dans lequel peut être placé le stérilet et qui est pourvu de moyens pour chasser le stérilet, caractérisé en ce qu'il reçoit le stérilet complet, avec son élément hélicoïdal (8) et muni d'un élément transversal (1), ce dernier étant disposé par ses extrémités dans des fentes (9) du petit tube (6) formant l'instrument.

# Fig.1

# Fig.2

# Fig.3